# EUROPEAN PATENT SPECIFICATION

(11) **EP 1 993 991 B1**
(45) Date of publication and mention of the grant of the patent: **22.09.2010**
(21) Application number: 07713090.4
(22) Date of filing: 01.03.2007
(51) Int. Cl.: C07C 209/08, C07C 211/19, C07C 17/16, C07C 22/08, C07C 211/27

(54) **PROCESS FOR THE PREPARATION OF 3,5-BIS (TRIFLUOROMETHYL)-N-METHYLBENZYLAMINE**
VERFAHREN ZUR HERSTELLUNG VON 3,5-BIS(TRIFLUORMETHYL)-N-METHYLBENZYLAMIN
PROCEDE POUR LA PREPARATION DE 3,5-BIS (TRIFLUOROMETHYL)-N-METHYLBENZYLAMINE

(30) Priority: 17.03.2006 IT MI20060488
(43) Date of publication of application: 26.11.2008
(73) Proprietor: MITENI S.p.A., 20138 Milan (IT)
(72) Inventor: MONDINI, Simonetta, I-36070 Trissino (VI) (IT); DALL'AVA, Mirco, I-36070 Trissino (VI) (IT); GUERRATO, Alfredo, I-36070 Trissino (VI) (IT)
(74) Representative: Trupiano, Federica
(86) International application number: PCT/IB2007/000492
(87) International publication number: WO 2007/107818

(56) References cited:
- GB-A- 2 282 807
- US-A- 5 760 248
- SINGER G.M. ET AL: J.MED.CHEM., vol. 29, 1986, pages 40-44, XP002446586

## Description

### FIELD OF THE INVENTION

The present invention concerns a process for the preparation of 3,5-bis(trifluoromethyl)-N-methyl-benzylamine by means of aN amino-dehalogenation reaction, more in particular starting from a 3,5-bis(trifluoromethyl)-benzyl halide, in particular from 3,5-bis(trifluoromethyl)-benzyl chloride and a new process for the preparation of this latter compound.

### TECHNICAL BACKGROUND

Syntheses of 3,5-bis(trifluoromethyl)-N-methyl-benzylamine starting from 3,5-bis(trifluoromethyl)-benzaldehyde are known, as described in WO2005/000821 and GB2282807; in the latter document the yields reported are around 50%. WO2004/041163, on the other hand, describes the synthesis starting from 3,5-bis(trifluoromethyl)-benzylamine passing through three reactions: formation of the derivative tert-butylcarbonate (BOC-), subsequent reaction of the latter with iodomethane in the presence of sodium hydride and deprotection of the 3,5-bis(trifluoromethyl)-N-Boc-N-methylbenzylamine, the overall reaction yield being equal to 75%.

It is known that the amino-dehalogenation reactions of halo-substrates with primary, amines do not normally provide interesting results since the secondary amines that are produced are more reactive than the starting primary amines and compete with the latter in the amino-dehalogenation reaction with consequent abundant formation of bis-derivatives and beyond, up to quaternary ammonium salt (see for example March's, Advanced Organic Chemistry; 2001, 5th edition, 499).

### SUMMARY OF THE INVENTION

The object of the present invention is to provide an alternative synthesis for preparation of the 3,5-bis(trifluoromethyl)-N-methyl-benzylamine which produces good yields without the formation of undesired by-products.

It has now surprisingly been found out that it is possible to prepare 3,5-bis(trifluoromethyl)-N-methyl-benzylamine via an amino-dehalogenation reaction starting from a 3,5-bis(trifluoromethyl)-benzyl halide in the presence of methylamine, with optimal yields and without the formation of by-products.

### DETAILED DESCRITPION OF THE INVENTION

Thus, according to one of its embodiments, the invention concerns a process for the preparation of 3,5-bis(trifluoromethyl)-N-methyl-benzylamine of formula (I) 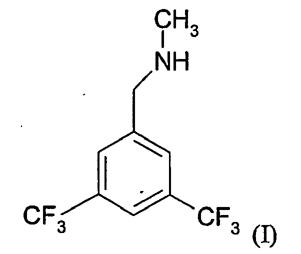 which comprises reacting a 3,5-bis(trifluoromethyl)-benzyl halide of formula (II), 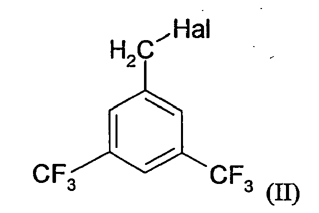 wherein Hal represents a halogen, with methylamine.

According to the present invention Hal represents one of the four halogens, advantageously bromine, chlorine and iodine, the chlorine being a particularly preferred halogen.

Methylamine can be used in any form available, in a gaseous or liquid form, for example in a 40% aqueous solution, which is commercially accessible.

According to a preferred embodiment of the invention, an excess of methylamine is used with respect to the compound of formula (II). The use of an excess of methylamine is important to prevent the formation of bis-derivatives and thus avoid the problems of the known technique, for example minimising the formation of the by-product having the following formula:

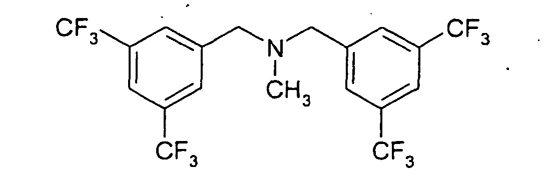

Indeed, it has surprisingly been found out, in fact, that to avoid formation of the above-mentioned bis-derivatives it is sufficient to use the methylamine in excess, advantageously in an excess of at least 5 times or more, with respect to the compound of formula (II).

According to a preferred embodiment of the invention, the reaction is performed in an appropriate solvent, advantageously of the polar type, preferably a polar solvent other than water, for example an alcohol. Methanol is a particularly preferred polar solvent.

The reaction is normally performed at a temperature between 0°C and the reflux temperature of the reaction mixture. By way of example, a temperature around 50-60°C allows the reaction to be performed at a reasonable speed: at said temperature, the reaction is generally complete in 2-10 hours.

The desired compound of formula (I) can be isolated according to the conventional techniques well known to a person skilled in the art and, if desired or necessary, further purified for example by distillation.

The starting 3,5-bis(trifluoromethyl)-benzyl halide of formula (II) is a known compound or can be prepared according to known methods.

As said, the preferred compound of formula (II) is 3,5-bis(trifluoromethyl)-benzyl chloride (Hal = Cl). Said compound can be prepared by chlorination of the corresponding benzyl alcohol, for example by reaction with thionyl chloride. 3,5-bis(trifluoromethyl)-benzyl chloride of formula (IIa), 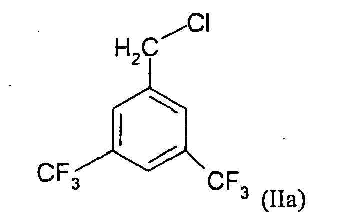 may be prepared by reacting the 3,5-bis(trifluoromethyl)-benzyl alcohol of formula (III) 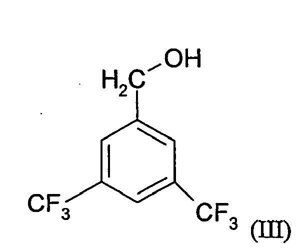 with thionyl chloride.

It is preferable to use an excess of thionyl chloride with respect to the stoichiometric quantity necessary for the chlorination, advantageously at least an excess of 1.5 moles with respect to the moles of the compound of formula (III).

The chlorination reaction described above can be performed with or without solvents; according to a particular embodiment of the present invention, said reaction is performed without solvents.

The reagents, compound of formula (III) and thionyl chloride, can be added to the reaction mixture in any order, i.e. first the thionyl chloride and then the compound of formula (III) can be metered in the reaction vessel or, vice versa, first the compound of formula (III) and then the thionyl chloride.

It is expedient to add to the reaction some dimethylformamide (DMF) which, acting as an additive-catalyst, promotes the desired reaction and prevents the formation of secondary products. To ensure successful reaction, it is sufficient to use a small amount of DMF, for example also in molar ratios of alcohol of formula (III) /DMF of around 1:0.25.

The reaction temperature can vary between - 10°C and the reflux temperature of the reaction mixture.

The reaction is complete in a few hours and the desired product of formula (IIa) can be isolated according to the conventional techniques.

Operating in the preferred conditions as described above, the required compound of formula (IIa) is obtained with excellent yields, generally above 95%.

Illustrative examples of the reaction are provided in the experimental section of the present description.

According to another of its embodiments, the invention concerns a process for the preparation of 3,5-bis(trifluoromethyl)-N-methyl-benzylamine of formula (I) 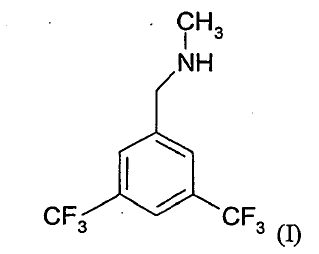 which comprises:
(a) reacting the 3,5-bis(trifluoromethyl)-benzyl alcohol of formula (III) with thionyl chloride to give 3,5-bis(trifluoromethyl)-benzyl chloride of formula (IIa);
(b) reacting said 3,5-bis(trifluoromethyl)-benzyl chloride of formula (IIa) with methylamine in an appropriate solvent; and
(c) optionally isolating the compound of formula (I) according to the conventional methods.

The invention will now be illustrated purely by way of non-limiting example.

### EXPERIMENTAL SECTION

### Example 1

### Preparation of the 3,5-bis(trifluoromethyl)-benzyl chloride

922.3 g of thionyl chloride (7.752 moles) are placed in a two-litre glass flask provided with mechanical stirring, thermometer, refrigerant, heated metering funnel and brine bath at 0°/+5°C, and 152.0 g of anhydrous DMF are added in portions, under stirring. The solution is cooled at -5/0°C by means of a brine bath. 3,5-bis(trifluoromethyl)-benzyl alcohol (1015.0 g, 4.072 moles) are added, keeping the mixture under stirring at a controlled temperature <10°C. The metering is completed in approximately 2 hours 45 minutes. The mixture is left under stirring at 20°C a further 5 hours to complete the reaction. The GC control at this point indicates a conversion >99.5% and a titer in 3,5-bis(trifluoromethyl)-benzyl chloride equal to 96.6%. The mass is brought to 50°C and the two phases are separated: 1352.8 g of yellow organic phase (lower phase) and 424.4 g of thionyl/DMF mixture are obtained. The organic phase is neutralised by two basic aqueous washing operations (10% solution of NaHCO3), in the cooled flask with a water/ice bath and after separation a further washing operation is performed with 10% NaOH at 50°C (1251.0 g). 1010.7 g of raw product are separated and purified by distillation.
Conversion (GC % area): >99.5%
Molar yield: 90%
GC titer of the 3,5-bis(trifluoromethyl)-benzyl chloride (% area): 99.2% min.

### Example 2

### Preparation of the 3,5-bis(trifluoromethyl)-N-methyl-benzylamine

600.5 g of 40% aqueous methylamine (7.9267 moles) and 400.9 g of methanol are placed in a two-litre glass flask provided with mechanical stirring, thermometer, water refrigerant, 250 ml metering funnel, oil heating bath, water scrubbing and Prahl head for concentration of the solvents. The mixture is brought to 50°C under stirring, while 200.2 g of 3,5-bis(trifluoromethyl)-benzyl chloride (0.7586 moles) in 100.7 g of methanol are added in the metering funnel. The chloride alcoholic solution is metered in 5.5 hours, and the reaction is monitored by GC halfway through metering and at the end. The Prahl head is fitted for removal of the methanol from the reaction mixture, maintaining a boiler temperature <80°C. 512.3 g of methanol and methylamine are distilled. After removal of the methanol, the organic phase is separated from the aqueous phase, transferring the mixture into a separator funnel (721.3 g). 176.0 g of 20% NaOH are added to the aqueous phase and it is extracted with 150 g of dichloromethane. All the organic phases are combined and washing is performed with 350.0 g of water. After evaporation of the solvent, 223.1 g of raw product are isolated and purified by distillation.
Conversion (GC % area): >99.5%
Molar yield: 84.0%
Titer of the 3,5-bis(trifluoromethyl)-N-methyl-benzylamine (% area): 99.75%.

## Claims

1. Process for the preparation of 3,5-bis(trifluoromethyl)-N-methylbenzylamine of formula (I) 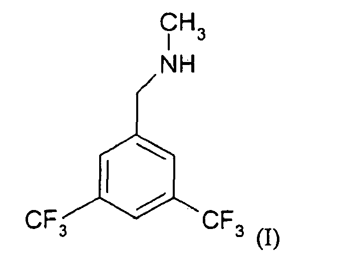 which comprises reacting a 3,5-bis(trifluoromethyl)-benzyl halide of formula (II), 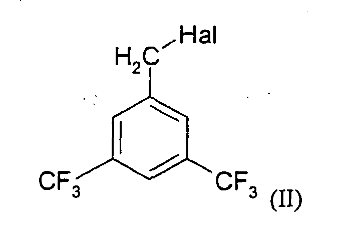 in which Hal represents a halogen, with methylamine.

2. Process as claimed in claim 1 in which Hal is a chlorine atom.

3. Process as claimed in one of the claims 1 or 2 in which an excess of methylamine is used with respect to the compound of formula (II).

4. Process as claimed in claim 3 in which a molar ratio methylamine/compound of formula (II) of at least 5/1 is used.

5. Process as claimed in one of the claims from 1 to 4 is carried out in the presence of a solvent.

6. Process as claimed in claim 5 in which said solvent is a polar solvent.

7. Process as claimed in claim 6 in which said polar solvent is an alcohol.

8. Process as claimed in claim 7 in which said alcohol is methanol.

9. Process for preparation of the 3,5-bis(trifluoromethyl)-N-methylbenzylamine of formula (I) 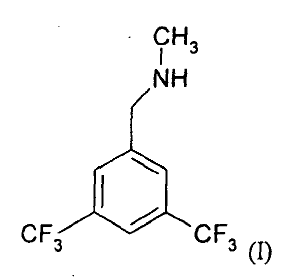 which comprises:
(a) reacting the 3,5-bis(trifluoromethyl)-benzyl alcohol of formula (III) with thionyl chloride in the presence of dimethylformamide, to give the 3,5-bis(trifluoromethyl)-benzyl chloride of formula (IIa);
(b) reacting said 3,5-bis(trifluoromethyl)-benzyl chloride of formula (IIa) with methylamine in a polar solvent; and
(c) optionally isolating the compound of formula (I).

10. Process as claimed in claim 9 in which said polar solvent is an alcohol.

11. Process as claimed in claim 10 in which said alcohol is methanol.

## Patentansprüche

1. Verfahren zur Herstellung von 3,5-Bis(Trifluormethyl)-N-Methylbenzylamin
mit der Formel (I) 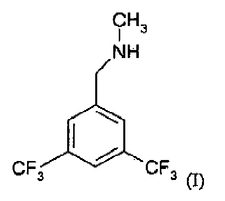 umfassend die Umsetzung eines 3,5-Bis(Trifluormethyl)-Benzylhalogenids mit der Formel (II), 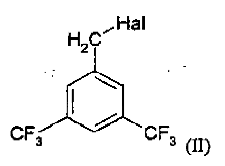 wobei Hal ein Halogen darstellt, mit Methylamin.

2. Das Verfahren gemäß Anspruch 9, wobei Hal ein Chloratom darstellt.

3. Das Verfahren gemäß Anspruch 1 oder 2, wobei ein Überschuss an Methylamin in Bezug auf die Verbindung mit der Formel (II) verwendet wird.

4. Das Verfahren gemäß Anspruch 3, wobei ein molares Verhältnis von Methylamin/Verbindung mit der Formel (II) von mindestens 5/1 verwendet wird.

5. Das Verfahren gemäß einem der Ansprüche 1 bis 4, wobei dieses in Anwesenheit eines Lösungsmittels durchgeführt wird.

6. Das Verfahren gemäß Anspruch 5, wobei das Lösungsmittel ein polares Lösungsmittel ist.

7. Das Verfahren gemäß Anspruch 6, wobei das polare Lösungsmittel ein Alkohol ist.

8. Das Verfahren gemäß Anspruch 7, wobei der Alkohol Methanol ist

9. Verfahren zur Herstellung von 3,5-Bis(Trifluormethyl)-N-Methylbenzylamin mit der Formel (I) 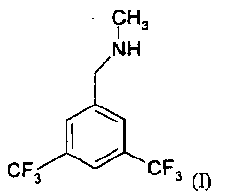 umfassend:
(a) die Umsetzung von 3,5-Bis(Trifluormethyl)-Benzylalkohol mit der Formel (III) mit Thionylchlorid in Anwesenheit von Dimethylformamid, um 3,5-Bis(Trifluormethyl)-Benzylchlorid mit der Formel (IIa) zu erhalten;
(b) die Umsetzung von 3,5-Bis(Trifluormethyl)-Benzylchlorid mit der Formel (IIa) mit Methylamin in einem polaren Lösungsmittel; und
(c) optional, Isolieren der Verbindung der Formel (I).

10. Das Verfahren gemäß Anspruch 9, wobei das polare Lösungsmittel ein Alkohol ist.

11. Das Verfahren gemäß Anspruch 10, wobei der Alkohol Methanol ist.

## Revendications

1. Procédé pour la préparation de 3,5-bis(trifluorométhyl)-N-méthylbenzylamine de formule (I) 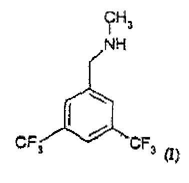 qui comprend la réaction d'un halogénure de 3,5-bis(trifluorométhyl)-benzyle de formule (II), 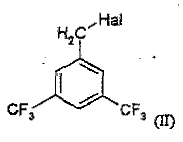 dans laquelle Hal représente un halogène, avec une méthylamine.

2. Procédé selon la revendication 1, dans lequel Hal est un atome de chlore.

3. Procédé selon l'une quelconque des revendications 1 ou 2, dans lequel un excès de methylamine est utilisé par rapport au composé de formule (II).

4. Procédé selon la revendication 3, dans lequel un rapport molaire de méthylamine/composé de formule (II) d'au moins 5/1 est utilisé.

5. Procédé selon l'une quelconque des revendications 1 à 4, qui est mis en oeuvre en présence d'un solvant.

6. Procédé selon la revendication 5, dans lequel ledit solvant est un solvant polaire.

7. Procédé selon la revendication 6, dans lequel ledit solvant polaire est un alcool.

8. Procédé selon la revendication 7, dans lequel ledit alcool est le méthanol.

9. Procédé pour la préparation de la 3,5-bis(trifluorométhyl)-N-méthylbenzylamine de formule (I) 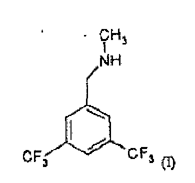 qui comprend :
(a) la réaction de l'alcool 3,5-bis(trifluorométhyl)-benzylique de formule (III) avec du chlorure de thionyle en présence de diméthylformamide, pour donner le chlorure de 3,5-bis(trifluorométhyl)-benzyle de formule (IIa) ;
(b) la réaction dudit chlorure de 3,5-bis(trifluorométhyl)-benzyle de formule (IIa) avec une méthylamine dans un solvant polaire ; et
(c) éventuellement l'isolement du composé de formule (I).

10. Procédé selon la revendication 9, dans lequel ledit solvant polaire est un alcool.

11. Procédé selon la revendication 10, dans lequel ledit alcool est le méthanol.
